(19)

Europäisches
Patentamt
European
Patent Office
Office européen
des brevets

(11) **EP 1 876 450 B1**

(12) **EUROPEAN PATENT SPECIFICATION**

(45) Date of publication and mention
of the grant of the patent:
**16.09.2009 Bulletin 2009/38**

(51) Int Cl.:
*G01N 33/80* (2006.01)  *G01N 33/96* (2006.01)
*G01N 1/44* (2006.01)  *G01N 33/94* (2006.01)

(21) Application number: **06013762.7**

(22) Date of filing: **03.07.2006**

(54) **A method for processing whole blood for analyte determination**

Verfahren zur Verarbeitung von Vollblut für analytische Zwecke

Procédé de traitement du sang complet pour la détermination d'un analyte

(84) Designated Contracting States:
**AT BE BG CH CY CZ DE DK EE ES FI FR GB GR
HU IE IS IT LI LT LU LV MC NL PL PT RO SE SI
SK TR**

(43) Date of publication of application:
**09.01.2008 Bulletin 2008/02**

(73) Proprietor: **SeBo GmbH
64711 Erbach (DE)**

(72) Inventor: **Seidel, Dietrich, Prof. Dr.
82340 Feldafing (DE)**

(74) Representative: **Weiss, Wolfgang et al
Weickmann & Weickmann
Patentanwälte
Postfach 86 08 20
81635 München (DE)**

(56) References cited:
**US-A- 4 716 119**

- **MARKUSZEWSKI ET AL: "Human red blood cells
targeted metabolome analysis of glycolysis cycle
metabolites by capillary electrophoresis using an
indirect photometric detection method"
JOURNAL OF PHARMACEUTICAL AND
BIOMEDICAL ANALYSIS, NEW YORK, NY, US,
vol. 39, no. 3-4, 15 September 2005 (2005-09-15),
pages 636-642, XP005039069 ISSN: 0731-7085**
- **RENNER S ET AL: "Analysis of metabolites of
glucose pathways in human erythrocytes by
analytical isotachophoresis" JOURNAL OF
CHROMATOGRAPHY, ELSEVIER SCIENCE
PUBLISHERS B.V. AMSTERDAM, NL, vol. 916, no.
1-2, 4 May 2001 (2001-05-04), pages 247-253,
XP004235554 ISSN: 0021-9673**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

The present invention refers to a method of processing whole blood by heat treatment. The method is particularly useful for preparing biological samples for analyte detection. Further, the invention refers to a cell-disintegrated whole blood comprising substantially quantitatively disintegrated cellular components.

The determination of analytes in samples from biological fluids often requires complicated and tedious pre-treatment procedures in order to remove cellular components from the fluid sample. For example, whole blood contains components, namely erythrocytes, leukocytes and thrombocytes. In order to determine analytes in a blood sample, these cellular components often have to be removed by pre-treatment procedures such as centrifugation, filtration, sedimentation and/or lysis by chemical reagents or mechanical treatment. These procedures, however, are often difficult to integrate into an automated test format. This holds also for a situation in which the target analytes are present in the cellular components, e.g. immuno-suppressiva in erythrocytes. In this case, the cellular components either are isolated or enriched by centrifugation and/or filtration prior to the addition of a lysis reagent or they are denatured by addition of a denaturing agent to the original sample, for example a mixture of $ZnSO_4$ and acetonitrile.

In Renner et al., J. Chromatography (2001), Vol. 916, pages 247-25, a method analysing metabolites of glucose pathways in human erythrocytes is disclosed. Erythrocytes are isolated from heparinized whole blood samples by centrifugation and in resuspended in PBS-buffer. For the preparation of erythrocyte lysates, the erythrocytes are pelleted, taken up in destilled water and boiled for 2 min.

In US 2001/051374 a control or calibration serum having a reduced tendency to turbidity formation upon reconstitution after previous lyophilization is disclosed.

The object of the present invention was to provide an improved method for processing biological fluids which does not have the disadvantages associated with prior art procedures.

Thus, a first aspect of the present invention is a method of processing a biological fluid which comprises cellular components, wherein the biological fluid is whole blood, wherein the fluid is subjected to a heat treatment under conditons

(i) to provide substantially quantitative disintegration of said cellular components and
(ii) not to cause substantial sedimentation, precipitation, denaturation, agglutination and gelation of fluid components, wherein the heat treatment is carried out under condition of temperature and time defined by an upper limit at a given temperature (indicated as value $t_{max}$) and by a lower time limit at a given temperature (indicated as value $t_{mln}$), and wherein, if the heat treatment is carried out for a time period longer than the $t_{max}$ value, gelation occurs, and wherein, if the heat treatment is carried out for a time period shorter than the $t_{min}$ value, incomplete disintegration occurs, and wherein the cell count in the biological fluid is reduced to 0.1 % or less.

A further aspect of the present invention is a cell-disintegrated whole blood obtainable by heat treatment of anticoagulant-treated whole blood according to the method as described above comprising substantially quantitatively disintegrated erythrocytes, leukocytes and thrombocytes, which is substantially free from sedimentation, precipitation, denaturation, agglutination and gelation products, and which is

(i) free from any particular components on microscopic observation with 100 x magnification; and
(ii) free from sediment after centrifugation for 10 min. at up to 3000 g.

Still a further aspect of the present invention is a method of determining an analyte in a whole blood sample, wherein the whole blood is processed as described above and the analyte is determined in the processed whole blood.

Surprisingly, the present inventor has found that a complete disintegration of cellular components, preferably cells or cell clusters from higher organisms, more preferably animal cells such as mammalian cells including human cells, and most preferably blood cells such as erythrocytes, leukocytes and/or thrombocytes in whole blood samples may be achieved by heat treatment under predetermined conditions of time and temperature. By means of this heat treatment, the cellular components contained in whole blood are disintegrated without substantial precipitation, denaturation, agglutination and/or gelation of fluid components.

The heat treatment may be carried out at a temperature of from 60-90°C, preferably from 60-75°C and more preferably from 65-70°C. The heat treatment is carried out for a time sufficient to achieve a desired disintegration at the chosen treatment temperature. Preferably, the heat treatment is carried out for a time of 5 sec to 1 min, more preferably from 10 sec to 40 sec. Especially preferred is a temperature of 70°C for 30-40, e.g. 35 sec.

In Table 1, suitable conditions for the heat treatment of a whole blood sample with a given erythrocyte count, optionally supplemented with organic solvents and/or plasma of blood group AB are shown. These temperature/time conditions are defined by an upper time limit (indicated as value tmax)at a given temperature and by a lower time limit (indicated as value tmin) at a given temperature. If the heat treatment is carried out for a time period longer than the tmax value, gelation occurs. If the heat treatment period is shorter than the tmin value, only incomplete disintegration occurs. If other fluids, e.g. organic solvents and/or aqueous fluids, e.g. plasma, are added to the sample before heat treatment, the values of tmax and tmin may vary as shown in Tables 2-6.

The gelation of the sample may be determined by an increase in viscosity. The completeness of disintegration may be determined by cell counting, e.g. in a Neubauer counting chamber, by microscopic inspection for particular components and/or by lack of sediment formation after centrifugation. In this context, it should be noted that about 95% of cellular blood components are represented by erythrocytes. Thus, the cell count in a blood sample is preferably determined by counting the erythrocytes.

By means of the present invention the cell count in the sample is preferably reduced to 0.1 % or less and more preferably to 0.01% or less of the original value. For example, when subjecting a sample with $5.10^6$ erythrocytes per $\mu$l to heat treatment, the cell count is preferably reduced to $5 \cdot 10^3$ cells or less per $\mu$l (cf. Example 1), more preferably to 500 cells or less per $\mu$l. Most preferably, the sample is free from detectable cells. The absence of particular components may also be determined by light-microscopic observation, e.g. up to 100x magnification, and/or by centrifugation for 10 min at up to 3000g, preferably at up to 7400g.

The heat treatment may be carried out when the fluid is kept static, e.g. while the fluid is in a reaction vessel. In a preferred embodiment, however, particularly for automated operations, the heat treatment may be carried out while the fluid is in flow, e.g. while the fluid is passed through a conduit. The heat treatment in a flowing system may be carried out while passing the sample fluid through a heated conduit, e.g. a capillary conduit, preferably with an inner diameter of about 0.1-0.8 mm, for example of about 0.5 mm with a predetermined flow rate, wherein the conduit has a predetermined length in order to achieve the desired residence time within the heated conduit. The heating may be carried out by any suitable means and may comprise e.g. inductive heating such as microwave treatment, for example as described in US 6,605,454, convective heating, resistive heating and/or heating by laser excitation.

The biological fluid is whole blood, such as venous, arterial or capillary blood, particularly anticoagulant-treated whole blood, e.g. EDTA-, citrate-, or heparin-treated whole blood. For example, a sample may be taken with an anticoagulant containing blood withdrawal device and directly subjected to further procesing as described below.

The sample volume may be varied broadly, e.g. in the range of 1 nl or more, preferably 10 nl or more and up to 1 ml. Thus, the method is suitable for miniaturized applications, e.g. microfluidic devices on chip format, nano LC-MS analysis etc.

The method of the present invention does not require sedimentation and/or precipitation steps such as centrifugation and/or the addition of chemical lysis and/or disintegration reagents. Thus, the heat treatment is preferably carried out without previous removal and/or lysis of cellular components. The method may be carried out in any suitable device, e.g. a single-use device or a reusable device. Preferably, the method is an automated procedure, which may be carried out in an integrated device, i.e. a device into which the fluid sample is transferred without pretreatment, particularly without removal and/or lysis of cellular components. Within the device, the sample is preferably directly subjected to the treatment without prior removal and/or a lysis of cellular components. After treatment, subsequent steps, e.g. an analyte determination may be carried out. Most preferably, the heat treatment is carried out with a substantially native sample, e.g. a sample comprising substantially intact cellular components.

The method of the present invention may include the addition of further fluid to the biological fluid before and/or after processing. The further fluid may be an organic solvent, preferably in an amount of up to 20% (vol/vol), more preferably in an amount of up to 10% (vol/vol) based on the volume of the biological fluid. The organic solvent is preferably selected from water-miscible solvents such as methanol, ethanol, acetonitrile, dimethylsulfoxide, and combinations thereof. The addition of organic solvents may have an influence on the temperature/time conditions for the heat treatment as shown in Tables 2 and 3.

Preferably, the further fluid does not substantially effect lysis of cellular components. More preferably, the further fluid is an aqueous fluid, e.g. an aqueous buffer solution or a further biological fluid, preferably having a ionic strength corresponding to 0.5-1.4% NaCl, more preferably 0.7-1.2% NaCl and most preferably about 0.9% NaCl. Preferably, the aqueous fluid is a biological fluid without cellular components such as plasma. More preferably, the plasma is plasma of the blood group AB. The further aqueous fluid may comprise an organic solvent, e.g. in an amount of up to 20% (vol/vol), preferably up to 10% (vol/vol) based on the volume of the second biological fluid such as methanol, ethanol, acetonitrile, dimethylsulfoxide and/or combinations thereof. The second biological fluid is preferably added in a volume ratio of 5:1 to 1:10 based on the volume of the first biological fluid. Preferably, the second fluid is added before the heat treatment. The addition of the second fluid may influence the suitable temperature/time conditions for the heat treatment step as shown in Tables 4-6. Surprisingly, it was found that addition of AB plasma, optionally with an organic solvent, actually increases the suitable treatment time range at a given temperature.

The further fluid may be a standardisation and/or calibrator fluid comprising a predetermined amount of at least one standardisation and/calibrator compound. The addition of standardisation and/or calibrator compounds is particularly suitable if the heat-treated biological fluid is further analysed by means of chromatographic, spectrometric and/or spectroscopic methods. The standardisation and/or calibrator compounds may be analyte analogues which contain stable isotopes such as $^2$H and/or $^{13}$C and thus may be detected by mass spectrometry.

The method also may include the addition of a marker/staining compound for lipids, proteins, peptides, nucleic acids and carbohydrates to the biological fluid before and/or after processing.

A further aspect of the present invention refers to the use of a composition comprising AB plasma, an organic solvent in an amount of up to 20% (vol/vol), preferably up to 10% (vol/vol) based on the volume of the plasma, such as methanol, ethanol, acetonitrile and/or dimethylsulfoxide and a predetermined amount of at least one standardisation and/or calibrator compound, in combination with the heat treatment procedure as described above.

Still a further aspect of the present invention refers to cell-disintegrated whole blood as described above. This fluid represents a novel matrix which is particularly suitable for clinical testing. The processed fluid is stable at least 1 week, preferably at least 2 weeks at 4°C and/or at least 1 day, preferably at least 5 days, at 25°C. Thus, the handling of the processed fluid is much more uncomplicated compared to a non-treated whole blood sample. The term "stable" particularly means that sedimentation does not occur.

The cell - disintegrated whole blood comprises substantially quantitatively disintegrated blood cells as described above. The processed fluid is substantially free from precipitation, denaturation, agglutination and/or gelation products. The invention refers to a processed whole blood which

(i) is free from any particular components on microscopic observation with 100 x magnification; and
(ii) is free from sediment after centrifugation for 10 min. at up to 3000g, preferably at up to 7400 g.

The processed fluid preferably has a ionic strength corresponding to 0.5-1.4% NaCl, more preferably 0.7-1.2% NaCl and most preferably a substantially physiological salt concentration. The processed fluid may be free from added disintegration and/or lysis reagents and/or detergents. On the other hand, the processed fluid may also comprise organic solvents and/ or added aqueous fluid such as plasma of blood group AB as described above. The processed fluid is processed whole blood.

The present invention also refers to a method of determining an analyte in a whole blood sample which has been subjected to a heat treatment as described above. The analyte may be any analyte which may be detected in whole blood, e.g. a biological compound such as a nucleic acid, a polypeptide, peptide, lipid, sugar, hormone, metabolite, etc. On the other hand, the analyte may be a non-biological compound, e.g. a pharmaceutical compound. In a preferred embodiment, the analyte is an immosuppressive drug, such as cyclosporin, rapamycin or tacrolimus or related compounds.

The analyte determination in the processed whole blood may be carried out according to any known method. For example, the analyte determination may be carried out acording to chemical, biochemical and/or physicochemical methods and may comprise a hybridization reaction, an immunological reaction, an enzymatic reaction, e.g. a nucleic acid amplification, a chromatographic analysis, a spectrometric analysis, such as a mass-spectrometric or a NMR analysis and/or a spectroscopic analysis. In an especially preferred embodiment, the invention refers to a method of determining an immunosuppressive agent in a whole blood sample, wherein the whole blood is processed by a heat treatment as described above and the immunosuppressive agent is determined in the processed whole blood according to standard methods, e.g. by mass-spectrometric methods.

In a further preferred embodiment, the analyte is a clinical-chemical parameter, e.g. a clinical-chemical parameter associated with an inborn metabolic disorder, e.g. phenylketonuria. In this embodiment, the sample is preferably a capillary blood sample which may be obtained from newborns.

In a still further preferred embodiment, the method is suitable for processing blood samples from non-human animals, preferably mice, guinea pigs and rats. For example, the samples may be taken by automated systems and directly processed as described above. A preferred automated system is the Accu Sampler ® from DiLab®.

Further, the present invention is explained in more detail by the following examples.

Example 1: Heat treatment of blood samples (static system)

A glass-capillary (55 mm length x 0.5 mm inner diameter) is filled with about 10 $\mu$l of a blood sample (erythrocytes: 5.18 x 10$^6$/$\mu$l; hemoglobin: 17.5 g/dl; hematocrite 50.1 %), sealed at one end with plasticine and heated in a thermostated water-bath at a given temperature for a given time. At the end of the heating process the glass-capillary is immediately immersed in an icebath (4°C). The plasticine sealing is cut off, the glass-capillary is emptied and the treated blood sample is further investigated for gelation and completeness of blood cell disintegration. The parameter $t_{max}$ is defined as the heating time [sec] at which gelation of the sample occurs minus 1 second. The parameter $t_{min}$ is defined as the minimal heating time at which no erythrocytes are detected using a Neubauer counting chamber.

Example 2: Erythrocyte count

To 10 $\mu$l of whole blood or treated blood 990 $\mu$l of Hayemsch-solution (Merck KGaA, Darmstadt, Germany) are added. The mixture is vortexed and an aliquot is introduced into a Neubauer counting chamber. The erythrocytes present in 5 defined squares are counted using a microscope and a magnification of 100.

Calculation:

$$\text{Erythrocytes/}\mu\text{l (sample)} = \frac{\text{Number of erythrocytes counted x 100}}{0.2\ \text{mm}^2\,\text{x}\ 0.1\ \text{mm}}$$

The results are shown in the following Tables 1-6.

Table 1: Heat treatment of whole blood (erythrocytes: $5.18 \times 10^6/\mu\text{l}$; hemoglobin: 17.5 g/dl; hematocrite 50.1 %).

| Temperature [°C] | $t_{max}$ [sec] | $t_{min}$ [sec] |
|---|---|---|
| 90 | 3 | 3 |
| 85 | 5 | 4 |
| 80 | 9 | 5 |
| 75 | 23 | 9 |
| 70 | 48 | 21 |
| 65 | 242 | 33 |
| 60 | 802, | 349 |

Table 2: Heat-treatment of whole blood containing 5 vol % of methanol

| Temperature [°C] | $t_{max}$ [sec] | $t_{min}$ [sec] |
|---|---|---|
| 80 | 6 | 6 |
| 75 | 13 | 7 |
| 70 | 42 | 11 |
| 65 | 169 | 18 |
| 60 | 646 | 21 |

Table 3: Heat-treatment of whole blood containing 5 vol % of acetonitrile

| Temperature [°C] | $t_{max}$ [sec] | $t_{min}$ [sec] |
|---|---|---|
| 80 | 6 | 5 |
| 75 | 10 | 9 |
| 70 | 21 | 10 |
| 65 | 49 | 11 |
| 60 | 184 | 32 |

Table 4: Heat-treatment of a 1:1 mixture of whole blood and AB-plasma

| Temperature [°C] | $t_{max}$ [sec] | $t_{min}$ [sec] |
|---|---|---|
| 80 | 15 | 8 |

(continued)

| Temperature [°C] | $t_{max}$ [sec] | $t_{min}$ [sec] |
|---|---|---|
| 75 | 31 | 9 |
| 70 | 65 | 41 |
| 65 | 412 | 66 |

Table 5: Heat-treatment of a 1:1 mixture of whole blood and AB-plasma containing 5 vol % of methanol

| Temperature [°C] | $t_{max}$ [sec] | $t_{min}$ [sec] |
|---|---|---|
| 80 | 5 | 3 |
| 75 | 13 | 3 |
| 70 | 38 | 3 |
| 65 | 126 | 8 |
| 60 | 693 | 28 |

Table 6: Heat-treatment of a 1:1 mixture of whole blood and AB-plasma containing 2.5, 5 or 10 vol % acetonitrile

| Temperature [°C] | $t_{max}$[sec] | | | $t_{min}$ [sec] | | |
|---|---|---|---|---|---|---|
| | acetonitrile vol % | | | acetonitrile vol % | | |
| | 2.5 | 5 | 10 | 2.5 | 5 | 10 |
| 80 | 11 | 5 | 3 | 5 | 4 | 1 |
| 75 | 23 | 11 | 4 | 3 | 2 | 1 |
| 70 | 41 | 24 | 5 | 4 | 5 | 1 |
| 65 | 171 | 53 | 17 | 10 | 12 | 1 |
| 60 | 753 | 281 | 35 | 11 | 33 | 2 |

Example 3: Heat treatment of a blood sample (Flow system)

For the treatment of a blood sample (e.g. 10 μl) using a heated stainless-steel capillary with the dimension of 0.5 mm internal diameter and 300 mm in length, the heating time at a given temperature can be preadjusted via the flow-rate of a given test fluid such as 0.9 vol % NaCl solution.

For a temperature of 75°C the minimal capillary retention time $t_{min}$ of 9 sec (cf. Example 1, Table 1) is reached with a flow-rate of 466 μl/min and the maximal capillary retention time $t_{max}$ of 23 sec. (cf. Example 1, Table 1) is reached with a flow-rate of 183 μl/min. Thus, a preferred flow-rate for such a sample size and capillary configuration would be within these boundaries, e.g. amounting to approximately 325 μl/min. This flow-rate is also optimal for electrospray-ionisation in mass spectrometry.

Calculation:

$$\text{Flow rate [μl/min]} = \frac{\text{Volume Capillary [μl] + Volume Sample [μl]}}{t_{min} \text{ (or } t_{max}) \text{ [sec]}} \times 60$$

**Claims**

1. A method of processing a biological fluid which comprises cellular components, wherein the biological fluid is whole blood,
   wherein the fluid is subjected to a heat treatment under conditions

   (i) to provide substantially quantitative disintegration of said cellular components and
   (ii) not to cause substantial sedimentation, precipitation, denaturation, agglutination and gelation of fluid components,

   wherein the heat treatment is carried out under condition of temperature and time defined by an upper limit at a given temperature (indicated as value $t_{max}$) and by a lower time limit at a given temperature (indicated as value $t_{min}$), and wherein, if the heat treatment is carried out for a time period longer than the $t_{max}$ value, gelation occurs, and wherein, if the heat treatment is carried out for a time period shorter than the $t_{min}$ value, incomplete disintegration occurs, and wherein the cell count in the biological fluid is reduced to 0.1 % or less.

2. The method of claim 1 wherein the heat treatment is carried out at a temperature of from 60-90°C, preferably from 60-75°C.

3. The method of claim 1 or 2 wherein the heat treatment is carried out for a time of 5 sec to 1 min.

4. The method of any one of claims 1-3 wherein the heat treatment is carried out while the fluid is static.

5. The method of any one of claims 1-3 wherein the heat treatment is carried out while the fluid is in flow.

6. The method of any one of claims 1-5 wherein the heat treatment comprises inductive heating, convective heating, resistive heating and/or heating by laser excitation.

7. The method of any one of claims 1-6 wherein the biological fluid is anticoagulant - heated whole blood.

8. The method of any one of claims 1-7 which does not include

   (i) a sedimentation and/or precipitation step and/or
   (ii) an addition of disintegration reagents.

9. The method of any one of claims 1-8 which is carried out as an automatized procedure, preferably in an integrated device.

10. The method of any one of claims 1-9 wherein before and/or after processing a further fluid is added.

11. The method of claim 10 wherein the further fluid is an organic solvent in an amount of up to 20% (vol/vol) based on the volume of the biological fluid, such as methanol, ethanol, acetonitrile and/or dimethyl sulfoxide.

12. The method of claim 10 or 11 wherein the further fluid is an aqueous fluid without cellular components optionally comprising an organic solvent in an amount of up to 20% (vol/vol) based on the volume of the aqueous fluid.

13. The method of claim 12 wherein the further fluid is plasma, particularly plasma of blood group AB.

14. The method of any one of claims 10-13 wherein the further fluid is a standardisation and/or calibrator fluid comprising a predetermined amount of at least one standardization and/or calibrator compound.

15. The method of any one of the claims 10-14 wherein the further fluid is a composition comprising a plasma of blood group AB, a water-miscible organic solvent in an amount of up to 20% (vol/vol) based on the volume of the plasma, such as methanol, ethanol, acetonitrile, dimethyl sulfoxide and/or combinations thereof and a predetermined amount of at least one standardisation and/or calibrator compound.

16. Cell - disintegrated whole blood obtainable by heat treatment of anticoagulant - treated whole blood according to the method of any one of claims 1-7 comprising substantially quantitatively disintegrated erythrocytes, leukocytes and thrombocytes, which is substantially free from sedimentation, precipitation, denaturation, agglutination and gelation products, and which is

> (i) free from any particular components on microscopic observation with 100 x magnification; and
> (ii) free from sediment after centrifugation for 10 min. at up to 3000 g,

17. The cell - disintegrated whole blood of claim 16 which has a substantially physiological salt concentration.

18. A method of determining an analyte in a whole blood sample wherein the whole blood is processed according to the method of any one of claims 1-15 and the analyte is determined in the processed whole blood.

19. The method of claim 18 wherein the analyte is selected from biological compounds such as nucleic acids, polypeptides, peptides, lipids, sugars, hormones, metabolites etc. and pharmaceutical compounds.

20. The method of claim 18 or 19 wherein the analyte is an immunosuppressive drug, such as cyclosporin, rapamycin or tacrolimus.

21. The method any one of claims 18-20 wherein the determination comprises a hybridization reaction, an immunological reaction, an enzymatic reaction, a chromatographic analysis, a spectrometric analysis and/or spectroscopic analysis.

22. A method of determining an immunosuppressive agent in a whole blood sample wherein the whole blood is processed according to the method of any one of claims 1-15 and the immunosuppressive agent is determined in the processed whole blood.

23. A method of determining a clinical-chemical parameter in a whole blood sample from newborns, wherein the whole blood is processed according to the method of any one of claims 1-15 and the clinical-chemical parameter is determined in the processed whole blood.

24. The method of any one of claims 18-23 wherein the sample is processed and determined in an integrated device.

25. Use of a composition comprising a plasma of blood group AB, a water-miscible organic solvent in an amount of up to 20% (vol/vol) based on the volume of the plasma, such as methanol, ethanol, acetonitrile, dimethyl sulfoxide and/or combinations thereof and a predetermined amount of at least one standardisation and/or calibrator compound in a method according to any one of claims 1-15 or 18-24.

**Patentansprüche**

1. Verfahren zur Verarbeitung einer biologischen Flüssigkeit, die Zellbestandteile umfasst, wobei die biologische Flüssigkeit Vollblut ist, wobei die Flüssigkeit einer Wärmebehandlung unter Bedingungen unterzogen wird,

> (i) um im Wesentlichen quantitativen Aufschluss der Zellbestandteile bereitzustellen und
> (ii) um keine wesentliche Sedimentation, Präzipitation, Denaturierung, Agglutination oder Gelierung von Bestandteilen der Flüssigkeit zu verursachen,
> wobei die Wärmebehandlung unter Temperatur- und Zeitbedingungen durchgeführt wird, die durch eine obere Grenze bei einer vorgegebenen Temperatur (angegeben als Wert $t_{max}$) und durch eine untere Zeitgrenze bei einer vorgegebenen Temperatur (angegeben als Wert $t_{min}$) definiert sind, und wobei, wenn die Wärmebehandlung für eine Zeitdauer, die länger als der $t_{max}$-Wert ist, durchgeführt wird, Gelierung auftritt, und wobei, wenn die Wärmebehandlung für eine Zeitdauer, die kürzer als der $t_{min}$-Wert ist, durchgeführt wird, unvollständiger Aufschluss auftritt, und wobei die Zellzahl in der biologischen Flüssigkeit auf 0,1 % oder weniger reduziert wird.

2. Verfahren nach Anspruch 1, wobei die Wärmebehandlung bei einer Temperatur von 60-90°C, bevorzugt von 60-75°C durchgeführt wird.

3. Verfahren nach Anspruch 1 oder 2, wobei die Wärmebehandlung für eine Zeitspanne von 5 s bis 1 min durchgeführt wird.

4. Verfahren nach einem der Ansprüche 1-3, wobei die Wärmebehandlung durchgeführt wird, während die Flüssigkeit stationär ist.

5. Verfahren nach einem der Ansprüche 1-3, wobei die Wärmebehandlung durchgeführt wird, während die Flüssigkeit fließt.

6. Verfahren nach einem der Ansprüche 1-5, wobei die Wärmebehandlung Induktionserwärmung, Konvektionserwärmung, Widerstandserwärmung und/oder Erwärmung durch Laseranregung umfasst.

7. Verfahren nach einem der Ansprüche 1-6, wobei die biologische Flüssigkeit mit Antikoagulans behandeltes Vollblut ist.

8. Verfahren nach einem der Ansprüche 1-7, welches nicht umfasst

> (i) einen Sedimentations- und/oder Präzipitationsschritt und/oder
> (ii) Zugabe von Aufschlussreagenzien.

9. Verfahren nach einem der Ansprüche 1-8, welches als automatisiertes Verfahren, bevorzugt in einer integrierten Vorrichtung, durchgeführt wird.

10. Verfahren nach einem der Ansprüche 1-9, wobei vor und/oder nach der Verarbeitung eine weitere Flüssigkeit zugegeben wird.

11. Verfahren nach Anspruch 10, wobei die weitere Flüssigkeit ein organisches Lösungsmittel in einer Menge von bis zu 20% (Vol/Vol) ist, bezogen auf das Volumen der biologischen Flüssigkeit, wie z.B. Methanol, Ethanol, Acetonitril und/oder Dimethylsulfoxid.

12. Verfahren nach Anspruch 10 oder 11, wobei die weitere Flüssigkeit eine wässrige Flüssigkeit ohne Zellbestandteile ist, die gegebenenfalls ein organisches Lösungsmittel in einer Menge von bis zu 20% (Vol/Vol), bezogen auf das Volumen der wässrigen Flüssigkeit, umfasst.

13. Verfahren nach Anspruch 12, wobei die weitere Flüssigkeit Plasma, insbesondere Plasma der Blutgruppe AB, ist.

14. Verfahren nach einem der Ansprüche 10-13, wobei die weitere Flüssigkeit eine Standardisierungs- und/oder Kalibrierungsflüssigkeit ist, die eine festgesetzte Menge mindestens einer Standardisierungs- und/oder Kalibrierungsverbindung umfasst.

15. Verfahren nach einem der Ansprüche 10-14, wobei die weitere Flüssigkeit eine Zusammensetzung ist, die ein Plasma der Blutgruppe AB, ein wassermischbares organisches Lösungsmittel in einer Menge von bis zu 20% (Vol/Vol), bezogen auf das Volumen des Plasmas, wie z.B. Methanol, Ethanol, Acetonitril, Dimethylsulfoxid und/ oder Kombinationen davon und eine festgesetzte Menge mindestens einer Standardisierungs- und/oder Kalibrierungsverbindung umfasst.

16. Vollblut mit aufgeschlossenen Zellen, erhältlich durch Wärmebehandlung von mit Antikoagulans behandeltem Vollblut gemäß dem Verfahren nach einem der Ansprüche 1-7, das im Wesentlichen quantitativ aufgeschlossene Erythrozyten, Leukozyten und Thrombozyten umfasst, das im Wesentlichen frei von Sedimentations-, Präzipitations-, Denaturierungs-, Agglutinations- und Gelierungsprodukten ist und das

> (i) bei mikroskopischer Beobachtung mit 100x Vergrößerung frei von irgendwelchen bestimmten Bestandteilen; und
> (ii) nach der Zentrifugation für 10 min bei bis zu 3000 g frei von Sediment ist.

17. Vollblut mit aufgeschlossenen Zellen nach Anspruch 16, das eine im Wesentlichen physiologische Salzkonzentration hat.

18. Verfahren zur Bestimmung eines Analyten in einer Vollblut-Probe, wobei das Vollblut gemäß dem Verfahren nach einem der Ansprüche 1-15 verarbeitet wird und der Analyt in dem verarbeiteten Vollblut bestimmt wird.

19. Verfahren nach Anspruch 18, wobei der Analyt aus biologischen Verbindungen, wie z.B. Nukleinsäuren, Polypeptiden, Peptiden, Lipiden, Zuckern, Hormonen, Metaboliten etc., und pharmazeutischen Verbindungen ausgewählt ist.

**20.** Verfahren nach Anspruch 18 oder 19, wobei der Analyt ein Immunsuppressivum, wie z.B. Cyclosporin, Rapamycin oder Tacrolimus ist.

**21.** Verfahren nach einem der Ansprüche 18-20, wobei die Bestimmung eine Hybridisierungsreaktion, eine immunologische Reaktion, eine enzymatische Reaktion, eine chromatographische Analyse, eine spektrometrische Analyse und/oder spektroskopische Analyse umfasst.

**22.** Verfahren zur Bestimmung eines Immunsuppressivums in einer Vollblut-Probe, wobei das Vollblut gemäß dem Verfahren nach einem der Ansprüche 1-15 verarbeitet wird und das Immunsuppressivum in dem verarbeiteten Vollblut bestimmt wird.

**23.** Verfahren zur Bestimmung eines klinisch-chemischen Parameters in einer Vollblut-Probe von Neugeborenen, wobei das Vollblut gemäß dem Verfahren nach einem der Ansprüche 1-15 verarbeitet wird und der klinisch-chemische Parameter in dem verarbeiteten Vollblut bestimmt wird.

**24.** Verfahren nach einem der Ansprüche 18-23, wobei die Probe in einer integrierten Vorrichtung verarbeitet und bestimmt wird.

**25.** Verwendung einer Zusammensetzung, die ein Plasma der Blutgruppe AB, ein wassermischbares organisches Lösungsmittel in einer Menge von bis zu 20% (Vol/ Vol), bezogen auf das Volumen des Plasmas, wie z.B. Methanol, Ethanol, Acetonitril, Dimethylsulfoxid und/oder Kombinationen davon und eine festgesetzte Menge mindestens einer Standardisierungs- und/oder Kalibrierungsverbindung umfasst, in einem Verfahren nach einem der Ansprüche 1-15 oder 18-24.

**Revendications**

**1.** Procédé de traitement d'un fluide biologique qui comprend des composants cellulaires, où le fluide biologique est du sang total, dans lequel ledit fluide est soumis à un traitement thermique dans certaines conditions

(i) pour obtenir une désintégration sensiblement quantitative desdits composants cellulaires et
(ii) pour ne pas provoquer de sédimentation, précipitation, dénaturation, agglutination et coagulation substantielles des composants du fluide,

dans lequel le traitement thermique est réalisé dans des conditions de température et de temps définies par une limite supérieure à une température donnée (indiquée comme la valeur $t_{max}$) et par une limite inférieure de temps à une température donnée (indiquée comme la valeur $t_{min}$) et dans lequel, si le traitement thermique est réalisé pendant un laps de temps supérieur à la valeur $t_{max}$, une coagulation se produit, et
dans lequel, si le traitement thermique est réalisé pendant un laps de temps inférieur à la valeur $t_{min}$, une désintégration incomplète se produit, et dans lequel la numération cellulaire dans le fluide biologique est réduite à 0,1 % ou moins.

**2.** Procédé selon la revendication 1, dans lequel le traitement thermique est réalisé à une température de 60-90°C, de préférence de 60-75°C.

**3.** Procédé selon la revendication 1 ou 2, dans lequel le traitement thermique est réalisé pendant un laps de temps de 5 sec à 1 min.

**4.** Procédé selon l'une quelconque des revendications 1-3, dans lequel le traitement thermique est réalisé pendant que le fluide est statique.

**5.** Procédé selon l'une quelconque des revendications 1-3, dans lequel le traitement thermique est réalisé pendant que le fluide s'écoule.

**6.** Procédé selon l'une quelconque des revendications 1-5, dans lequel le traitement thermique comprend un chauffage à induction, un chauffage à convection, un chauffage à résistance, et/ou un chauffage par excitation laser.

**7.** Procédé selon l'une quelconque des revendications 1-6, dans lequel le fluide biologique est du sang total traité avec un anticoagulant.

**8.** Procédé selon l'une quelconque des revendications 1-7, qui ne comprend pas :

(i) une étape de sédimentation et/ou précipitation et/ou
(ii) une addition de réactifs de désintégration.

**9.** Procédé selon l'une quelconque des revendications 1-8, qui est réalisé sous forme de procédure automatisée, de préférence dans un dispositif intégré.

**10.** Procédé selon l'une quelconque des revendications 1-9, dans lequel un autre fluide est ajouté, avant et/ou après le traitement.

**11.** Procédé selon la revendication 10, dans lequel l'autre fluide est un solvant organique, dans une quantité pouvant atteindre 20 % (en volume), sur la base du volume du fluide biologique, comme le méthanol, l'éthanol, l'acétonitrile et/ou le diméthylsulfoxyde.

**12.** Procédé selon les revendications 10 ou 11, dans lequel l'autre fluide est un fluide aqueux sans composants cellulaires, comprenant éventuellement un solvant organique dans une quantité pouvant atteindre 20 % (en volume), sur la base du volume du fluide aqueux.

**13.** Procédé selon la revendication 12, dans lequel l'autre fluide est du plasma, en particulier du plasma du groupe sanguin AB.

**14.** Procédé selon l'une quelconque des revendications 10-13, dans lequel l'autre fluide est un fluide de standardisation et/ou de calibration comprenant une quantité prédéterminée d'au moins un composé de standardisation et/ou de calibration.

**15.** Procédé selon l'une quelconque des revendications 10-14, dans lequel l'autre fluide est une composition comprenant du plasma du groupe sanguin AB, un solvant organique miscible dans l'eau, dans une quantité pouvant atteindre 20 % (en volume) sur la base du volume de plasma, comme le méthanol, l'éthanol, l'acétonitrile, le diméthylsulfoxyde et/ou des combinaisons de ceux-ci, et une quantité prédéterminée d'au moins un composé de standardisation et/ou de calibration.

**16.** Sang total à cellules désintégrées susceptible d'être obtenu par traitement thermique de sang total traité avec un anticoagulant, selon le procédé de l'une quelconque des revendications 1-17, comprenant des érythrocytes, leucocytes et thrombocytes sensiblement quantitativement désintégrés, qui est sensiblement dénué de produits de sédimentation, précipitation, dénaturation, agglutination et coagulation et qui est

(i) dénué de tout composant particulaire en observation microscopique avec un agrandissement de 100 fois ; et
(ii) dénué de sédiment après centrifugation pendant 10 minutes à un maximum de 3000 g.

**17.** Sang total à cellules désintégrées selon la revendication 16, ayant une concentration en sel sensiblement physiologique.

**18.** Procédé de détermination d'un analyte dans un échantillon de sang total, dans lequel le sang total est traité, selon le procédé de l'une quelconque des revendications 1-15 et l'analyte est déterminé dans le sang total traité.

**19.** Procédé selon la revendication 18, dans lequel l'analyte est choisi parmi des composés biologiques comme des acides nucléiques, des polypeptides, des peptides, des lipides, des sucres, des hormones, des métabolites etc, et des composés pharmaceutiques.

**20.** Procédé selon la revendication 18 ou 19, dans lequel l'analyte est un médicament immunosuppresseur, comme la cyclosporine, la rapamycine ou le tacrolimus.

**21.** Procédé selon l'une quelconque des revendications 18-20, dans lequel la détermination comprend une réaction d'hybridation, une réaction immunologique, une réaction enzymatique, une analyse chromatographique, une analyse spectrométrique et/ou une analyse spectroscopique.

22. Procédé de détermination d'un agent immunosuppresseur dans un échantillon de sang total, dans lequel le sang total est traité selon le procédé de l'une quelconque des revendications 1-15, et l'agent immunosuppresseur est déterminé dans le sang total traité.

23. Procédé de détermination d'un paramètre clinique-chimique dans un échantillon de sang total chez les nouveau-nés, dans lequel le sang total est traité, selon le procédé de l'une quelconque des revendications 1-15 et le paramètre clinique-chimique est déterminé dans le sang total traité.

24. Procédé selon l'une quelconque des revendications 18-25, dans lequel l'échantillon est traité et déterminé dans un dispositif intégré.

25. Utilisation d'une composition comprenant un plasma de groupe sanguin AB, un solvant organique miscible dans l'eau dans une quantité pouvant atteindre 20 % (en volume) sur la base du volume du plasma, comme le méthanol, l'éthanol, l'acétonitrile, le diméthylsulfoxyde et/ou des combinaisons de ceux-ci et une quantité prédéterminée d'au moins un composé de standardisation et/ou de calibration dans un procédé selon l'une quelconque des revendications 1-15 ou 18-24.

**REFERENCES CITED IN THE DESCRIPTION**

**Patent documents cited in the description**

- US 2001051374 A

- US 6605454 B

**Non-patent literature cited in the description**

- **Renner et al.** *J. Chromatography,* 2001, vol. 916, 247-25